# EUROPEAN PATENT APPLICATION

(11) **EP 4 635 502 A1**
(43) Date of publication of application: **22.10.2025**
(21) Application number: 23902736.0
(22) Date of filing: 13.12.2023
(51) Int. Cl.: A61K 31/737, A61K 36/03, A61P 11/00

(54) **SEAWEED-DERIVED SULPHATED POLYSACCHARIDE, PREPARATION METHOD THEREFOR, AND USE THEREOF**

(30) Priority: 16.12.2022 CN 202211624541
(71) Applicant: Shanghai Institute of Materia Medica, Chinese Academy of Sciences, Shanghai 201203 (CN)
(72) Inventor: DING, Kan, Shanghai 201203 (CN); LI, Jia, Shanghai 201203 (CN); ZANG, Yi, Shanghai 201203 (CN); LI, Saijuan, Shanghai 201203 (CN); ZHU, Anming, Shanghai 201203 (CN)
(74) Representative: Grünecker Patent- und Rechtsanwälte PartG mbB
(86) International application number: PCT/CN2023/138342
(87) International publication number: WO 2024/125540

(57) **Abstract**

The present invention belongs to the technical field of polysaccharides, and particularly relates to a *Sargassum fusiforme* sulfated polysaccharide, a preparation method therefor, and use thereof. The *Sargassum fusiforme* sulfated polysaccharide SPW-05-S2 contains 62% to 70% polysaccharide, 27% to 32% sulfate group, 0.5% to 1.5% acetyl and 2.0% to 4.5% protein, and has a weight average molecular weight of 121.77 kDa; the polysaccharide is mainly composed of mannose, glucuronic acid, galactose, xylose and fucose. In vivo experiments have shown that the polysaccharide can reduce bleomycin-induced pulmonary fibrosis, including restoring lung tissue morphology, reducing lung coefficient, and reducing the animal lung injury and expression of fibrosis phenotype-related proteins. It has no effect on cell growth activity in vitro, can significantly inhibit the expression of fibrosis-related proteins. Therefore, the polysaccharide has the potential to alleviate lung injury and is expected to become a candidate carbohydrate drug for the treatment of pulmonary fibrosis.

## Description

### Technical Field

The invention belongs to the technical field of polysaccharides, and particularly relates to a *Sargassum fusiforme* sulfated polysaccharide, a preparation method therefor and use thereof in preparing an anti-pulmonary fibrosis drug.

### Background Art

Pulmonary fibrosis is characterized by excessive deposition of extracellular matrix in the lung interstitium and destruction of normal parenchymal structure, leading to progressive loss of lung function. Various factors contribute to the development of pulmonary fibrosis, such as abnormal proliferation of fibroblasts, abnormal expression of myofibroblasts, damage of alveolar epithelium or endothelium, abnormal tissue repair, dysregulation of wound healing pathways, and excessive production and accumulation of extracellular matrix. The cause and pathogenesis of pulmonary fibrosis are unknown, which seriously affects the survival and quality of life of patients, and brings a heavy burden to patients' families and society. It is a chronic, progressive, and fatal lung disease. Therefore, It has important clinical significance to find a drug that can effectively treat pulmonary fibrosis.

The traditional Chinese medicine seaweed is the dried algae of *Sargassum pallidum* (Turn.) C. Ag or *Sargassum fusiforme* (Harv.) Setch., which belong to the Sargasaceae family. The former is commonly known as "big leaf seaweed" and the latter is commonly known as "small leaf *Sargassum fusiforme". Sargassum pallidum* is distributed along the Yellow Sea and Bohai Sea in Liaoning and Shandong. *Sargassum fusiforme* is distributed along the coasts of Liaoning, Shandong, Fujian, Zhejiang, Guangdong and other places. *Sargassum fusiforme* has the effects of clearing away heat and detoxifying, eliminating phlegm, softening and dispersing nodules, promoting diuresis and reducing swelling. It is often used for goiter, scrofula, testicular swelling and pain, and phlegm and edema. *Sargassum fusiforme* is rich in polysaccharides, which account for more than 50% of the dry weight of *Sargassum fusiforme.* As the main active substance in traditional Chinese medicine, polysaccharides play an important role in the exertion of its biological activity. Modern research shows that *Sargassum fusiforme* polysaccharides have a variety of biological activities, including antiviral, antitumor, anticoagulant, anti-atherosclerotic, anti-mutation, antioxidant and immune regulation, etc., which has attracted the attention of many researchers. However, there are few reports on natural polysaccharides with anti-pulmonary fibrosis activity in traditional Chinese medicine.

### Summary of the invention

The present invention adopts a simple and effective sulfated polysaccharide extraction process and method to obtain a sulfated-containing polysaccharide (named in this article: SPW-05-S2) from *Sargassum fusiforme* as raw material. In vitro experiments showed that SPW-05-S2 could improve the fibrotic phenotype of alveolar epithelial cells; and in vivo experiments proved that the polysaccharide could reduce lung damage and weaken pulmonary fibrosis in bleomycin-induced pulmonary fibrosis model mice, and it is expected to be developed into an anti-pulmonary fibrosis carbohydrate drug.

It is one object of the present invention to provide a *Sargassum fusiforme* sulfated polysaccharide.

It is the second object of the present invention to provide a method for preparing the *Sargassum fusiforme* sulfated polysaccharide.

It is the third object of the present invention to provide a pharmaceutical composition comprising the *Sargassum fusiforme* sulfated polysaccharide.

It is the fourth object of the present invention to provide a use of the *Sargassum fusiforme* sulfated polysaccharide or a pharmaceutical composition comprising the *Sargassum fusiforme* sulfated polysaccharide in the preparation of a drug for preventing and/or treating pulmonary fibrosis.

In order to achieve the above object of the present invention, the following technical solutions are particularly adopted:
In one aspect, the present invention provides a *Sargassum fusiforme* sulfated polysaccharide (SPW-05-S2), comprising 62% to 70% (e.g., 63%, 65%, 67%, 69%) of polysaccharide by weight, 27% to 32% (e.g., 28%, 29%, 30%, 31%) of sulfate group by weight, 0.5 % to 1.5% (e.g., 0.7%, 0.9%, 1.1%, 1.3%) of acetyl group by weight, and 2.0% to 4.5% (e.g., 2.5%, 3%, 3.5%, 4%) of protein by weight (the weight percentages here are calculated based on the weight of the *Sargassum fusiforme* sulfated polysaccharide);
The polysaccharide is mainly composed of mannose, glucuronic acid, galactose, xylose and fucose.

The structural unit of the polysaccharide is a main chain of 1→4-linked β-glucuronic acid and 1→2-linked α-mannose, with branches at the C-6 and C-3 positions of the α-mannose, and the branches are composed of galactose (1,3,6-β-galactose, 1,6-β-galactose and terminal-β-galactose), xylose (1,4-β-xylose and terminal-β-xylose) and fucose (1,2-α-fucose, 1,3-α-fucose, 1,4-α-fucose, 1,2,4-α-fucose and terminal-α-fucose) residues;
The sulfate group is mainly substituted at the C-4 position of 1,2-α-fucose, the C-2 position of 1,4-α-fucose, the C-4 position of terminal-β-galactose, and the C-4 position of part of terminal-β-xylose.

The weight average molecular weight of the *Sargassum fusiforme* sulfated polysaccharide is in the range of 5-1000 kDa, preferably 10-500 kDa, and more preferably 15-400 kDa.

Preferably, the molar ratio of mannose, glucuronic acid, galactose, xylose and fucose is 10-15: 1.5-4: 22-32: 5-9: 40-60, preferably 12.28: 2.60 : 27.59 : 7.06 : 50.47.

In some examples, the *Sargassum fusiforme* sulfated polysaccharide SPW-05-S2 contains 29.28% sulfate groups, 66.4% polysaccharides, 0.91% acetyl groups and 3.4% protein, and its weight average molecular weight is 121.77 kDa.

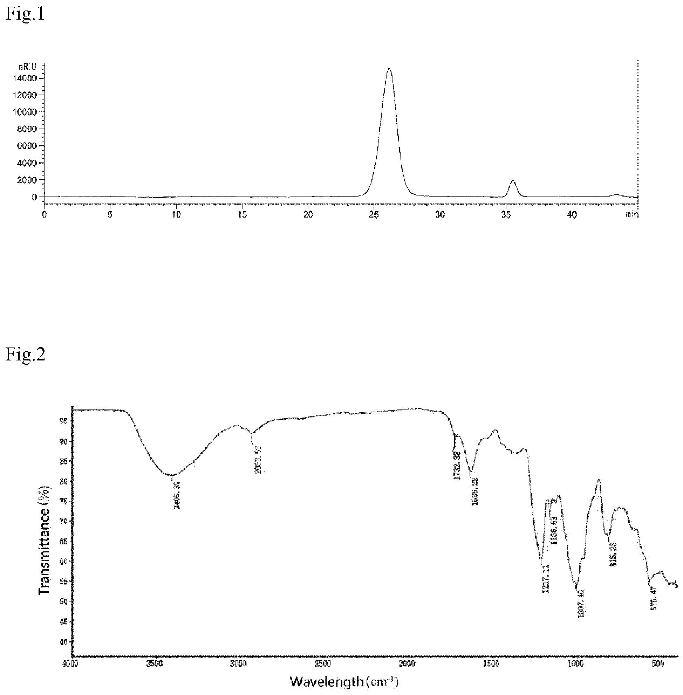

Preferably, the *Sargassum fusiforme* sulfated polysaccharide has a high-performance gel permeation chromatography (HPGPC) graph substantially as shown in FIG1.

Preferably, the *Sargassum fusiforme* sulfated polysaccharide has an infrared spectrum substantially consistent with the main stretching vibration absorption peak in Figure 2. In the infrared spectrum of the *Sargassum fusiforme* sulfated polysaccharide SPW-05-S2, the peak at 3308.69 cm⁻¹ is the O-H stretching vibration absorption peak, the peak at 2932.46 cm⁻¹ is the C-H stretching vibration absorption peak, the strong absorption peak at 1217.11 cm⁻¹ is the stretching vibration peak of O=S=O, and the peak at 1732.38 cm⁻¹ is the carboxyl C=O stretching vibration, indicating that the polysaccharide contains uronic acid.

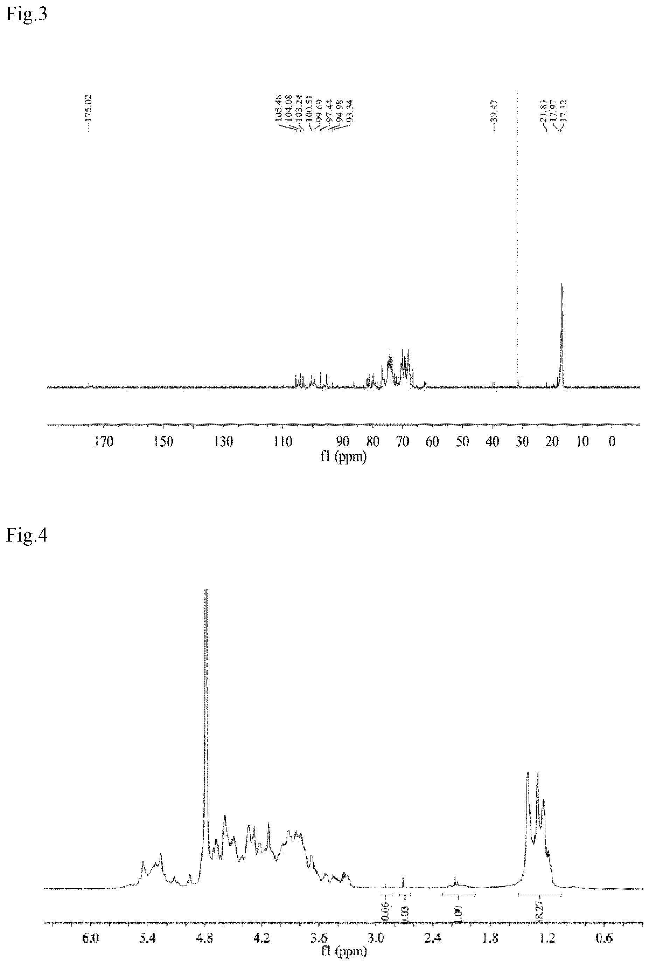

Preferably, the *Sargassum fusiforme* sulfated polysaccharide has a ¹³C NMR spectrum substantially consistent with the main signal values in Figure 3. In the ¹³C NMR spectrum of the *Sargassum fusiforme* sulfated polysaccharide, δ 105.48 - δ 104.08 signals are attributed to 1,3,6-β-galactose, 1,6-β-galactose, and terminal β-galactose. δ 103.24 - δ 100.51 signals are attributed to 1,2,3-α-mannose /1,2,6-α-mannose, 1,4-β-xylose and terminal-β-xylose and 1,4-β-glucuronic acid. δ 99.69 - δ 97.44 are attributed to 1,2-α-fucose, 1,4-α-fucose, 1,2,4-α-fucose and terminal-α-fucose. δ 93.44 is attributed to 1,3-α-fucose. δ 175.02 is attributed to carboxyl carbon of 1,4-β-glucuronic acid. δ 39.47 and δ 21.83 signal indicate that the polysaccharide contains acetyl groups. δ 17.97 and δ 17.12 are attributed to the C-6 of α-fucose.

Preferably, the *Sargassum fusiforme* sulfated polysaccharide of the present invention is extracted from *Sargassum fusiforme* produced in Zhejiang (classified as *Sargassum fusiformis* according to its shape and origin).

In another aspect, the present invention provides a method for preparing the *Sargassum fusiforme* sulfated polysaccharide (SPW-05-S2), comprising the following steps:
a. Sulfated polysaccharide extraction:
   The dried *Sargassum fusiforme* is subjected to boiling water extraction, the obtained extract is concentrated, dialyzed, re-concentrated, and centrifuged to remove the precipitate, the obtained supernatant is precipitated with alcohol, centrifuged and separated, and the obtained precipitate is washed and dried to obtain crude water-extracted *Sargassum fusiforme* sulfated polysaccharide;
   Preferably, the step a comprises: adding about 15 to 20 times the weight of water to the dried *Sargassum fusiforme,* raising the temperature to 100°C to perform boiling water extraction for 3 to 5 hours, extracting 7 to 9 times in total, combining and concentrating the extracts, dialyzing, re-concentrating, centrifuging to obtain a supernatant, adding about 5 to 10 times the volume of ethanol to the supernatant, centrifuging to obtain a precipitate, washing the precipitate with anhydrous ethanol and acetone for 3 to 6 times, and vacuum drying or freeze-drying to obtain a crude water-extracted *Sargassum fusiforme* sulfated polysaccharide;
   Further preferably, the step a comprises: adding about 20 times the weight of deionized water to the dried *Sargassum fusiforme,* raising the temperature to 100°C to perform boiling water extraction for 4 h, extracting 8 times in total, combining and concentrating the extracts, dialyzing, concentrating the dialyzed liquid, centrifuging to obtain a supernatant, adding about 5 times the volume of ethanol to the supernatant, centrifuging to obtain a precipitate, washing the precipitate with anhydrous ethanol and acetone alternately 3 times, drying in an oven, re-dissolving, and freeze-drying to obtain a crude water-extracted *Sargassum fusiforme* sulfated polysaccharide;
b. Sulfated polysaccharide purification:
   b1. Taking the crude water-extracted *Sargassum fusiforme* sulfated polysaccharide prepared in step a, dissolving in water and centrifuging. The supernatant was initially fractional purified by anion exchange column, sequentially eluted with water and 0.05 to 0.8 M NaCl solution, and the eluted fraction of about 0.5 M NaCl solution was collected to obtain sulfated polysaccharide SPW-05;
      Preferably, the step b1 comprises: taking the crude water-extracted *Sargassum fusiforme* sulfated polysaccharide prepared in step a, adding about 10 to 20 times the weight of water to dissolve, centrifuging, separating the supernatant through an anion exchange column, eluting with deionized water, 0.05 M, 0.1 M, 0.2 M, 0.3 M, 0.5 M, 0.6 M and 0.8 M NaCl solution in sequence, detecting with sulfuric acid-phenol, collecting and combining the eluate of about 0.5 M NaCl solution according to the elution curve, concentrating, centrifuging, dialyzing the supernatant, and freeze-drying to obtain the preliminarily purified sulfated polysaccharide SPW-05;
      Further preferably, the step b1 comprises: taking the crude water-extracted *Sargassum fusiforme* sulfated polysaccharide prepared in step a, adding about 10 times the weight of water to dissolve, centrifuging, separating the supernatant by DEAE Sepharose Fast Flow anion exchange column, eluting with deionized water, 0.05 M, 0.1 M, 0.2 M, 0.3 M, 0.5 M, 0.6 M and 0.8 M NaCl solution in sequence, detecting with sulfuric acid-phenol, collecting and combining the eluate of 0.5 M NaCl solution according to the elution curve, concentrating, centrifuging, dialyzing the supernatant, and freeze-drying to obtain the preliminarily purified sulfated polysaccharide SPW-05;
   b2. Dissolving the sulfated polysaccharide SPW-05 prepared in step b1 in about 0.01 to 1 times the weight of 0.2 M NaCl solution, centrifuging, and purifying the supernatant by gel chromatography column, eluting with 0.2 M NaCl solution, and collecting and combining the main component of the sulfated polysaccharide SPW-05-S2 according to the elution curve to obtain the sulfated polysaccharide SPW-05-S2;
Preferably, the step b2 comprises: dissolving the sulfated polysaccharide SPW-05 prepared in step b1 in about 0.01 times the weight of a 0.2 M NaCl solution, centrifuging, purifying the supernatant by a Sephacryl HR S-300 gel chromatography column, eluting with a 0.2 M NaCl solution, detecting with a sulfuric acid-phenol method, and collecting and combining the sulfated polysaccharide SPW-05-S2 according to the elution curve, concentrating, dialyzing, and freeze-drying this main component to obtain the sulfated polysaccharide SPW-05-S2.

In step a, the ethanol may be an aqueous solution of about 70% (v/v) or more, preferably an aqueous solution of about 85% (v/v) or more, and particularly an aqueous solution of about 95 % (v/v) or more.

Structural identification of sulfated polysaccharides: the structure of the *Sargassum fusiforme* sulfated polysaccharide SPW-05-S2 was determined by comprehensive analysis of its monosaccharide composition, methylation, infrared and nuclear magnetic resonance.

In another aspect, a pharmaceutical composition comprising a therapeutically effective amount of the *Sargassum fusiforme* sulfated polysaccharide as an active ingredient is provided. The composition may further include pharmaceutically acceptable excipients, such as carriers, excipients, adjuvants and/or diluents.

Another aspect of the present invention provides use of the *Sargassum fusiforme* sulfated polysaccharide or the pharmaceutical composition in preparing a drug for treating and/or preventing pulmonary fibrosis.

The pulmonary fibrosis is preferably bleomycin-induced pulmonary fibrosis.

In another aspect of the present invention, a method for reducing fibrotic phenotype, and preventing and/or treating pulmonary fibrosis is provided, and the method comprises administering a therapeutically effective amount of the *Sargassum fusiforme* sulfated polysaccharide or the pharmaceutical composition to a subject in need of such treatment.

The technical solution of the present invention has at least the following technical effects:
The present invention prepares a uniform sulfated polysaccharide component (molecular weight within a certain range (normal distribution) with a certain sugar residue connection mode and sugar composition) containing a specific sugar composition, sulfate group content, molecular weight and sulfate group substitution position through a specific extraction and separation method. In vivo experiments have shown that the polysaccharide SPW-05-S2 can reduce bleomycin-induced pulmonary fibrosis, including restoring lung tissue morphology, reducing lung coefficient, and reducing the animal lung injury and expression of fibrosis phenotype-related proteins. It has no effect on cell growth activity in vitro, can significantly inhibit the expression of fibrosis-related proteins, and has great application prospects in the prevention and/or treatment of pulmonary fibrosis candidate drugs.

The present invention has been described in detail above, but the above embodiments are only illustrative in nature and are not intended to limit the present invention. In addition, this application is not limited by any theory described in the above prior art or invention content or the following examples.

### BRIEF DESCRIPTION OF THE DRAWINGS

Figure 1 is a characteristic high-performance gel permeation chromatogram of *Sargassum fusiforme* sulfated polysaccharide SPW-05-S2;
Figure 2 is a characteristic infrared spectrum of *Sargassum fusiforme* sulfated polysaccharide SPW-05-S2;
Figure 3 is a characteristic ¹³C NMR spectrum of *Sargassum fusiforme* sulfated polysaccharide SPW-05-S2;
Figure 4 is a characteristic ¹H-QA (quantitative) NMR spectrum of *Sargassum fusiforme* sulfated polysaccharide SPW-05-S2;
Figure 5 is a bar graph of the effect of the *Sargassum fusiforme* sulfated polysaccharide SPW-05-S2 prepared in Preparation Example 1 on the mouse lung tissue morphology (A) and lung coefficient (B) in the bleomycin-induced mouse pulmonary fibrosis model (** represents P < 0.01);
Figure 6 is a representative image of hematoxylin-eosin staining (A) and Masson staining (B) of mouse lung tissue of *Sargassum fusiforme* sulfated polysaccharide SPW-05-S2 prepared in Preparation Example 1 in a bleomycin-induced mouse pulmonary fibrosis model;
Figure 7 is an immunoblot of Fibronectin, Collagen I and α-smooth muscle actin proteins by the *Sargassum fusiforme* sulfated polysaccharide SPW-05-S2 prepared in Preparation Example 1 in mouse lung tissue in the bleomycin-induced mouse pulmonary fibrosis model;
Figure 8 is a bar graph showing the effect of the *Sargassum fusiforme* sulfated polysaccharide SPW-05-S2 prepared in Preparation Example 1 on the viability of alveolar epithelial cells A549 using a CCK-8 cell proliferation and toxicity detection experiment (+ represents addition, - represents no addition);
Figure 9 is an immunoblot of the protein expression levels of E-cadherin, α-SMA, Collagen I and Fibronectin in TGF-β-induced alveolar epithelial cells A549 cells by the *Sargassum fusiforme* sulfated polysaccharide SPW-05-S2 prepared in Preparation Example 1 (+ represents addition, - represents no addition).

### DETAILED DESCRIPTION

In this document, all features or conditions defined in the form of numerical ranges or percentage ranges are only for brevity and convenience. Accordingly, the description of numerical ranges or percentage ranges should be deemed to have covered and specifically disclosed all possible secondary ranges and individual values within the range, especially integer values. For example, the range description of "1 to 8" should be deemed to have specifically disclosed all secondary ranges such as 1 to 7, 2 to 8, 2 to 6, 3 to 6, 4 to 8, 3 to 8, etc., especially secondary ranges defined by all integer values, and should be deemed to have specifically disclosed individual values such as 1, 2, 3, 4, 5, 6, 7, 8, etc. within the range. Unless otherwise specified, the above interpretation method applies to all contents of the entire present invention, regardless of whether the range is broad or not.

If the quantity or other numerical value or parameter is expressed as a range, a preferred range or a series of upper and lower limits, it should be understood that all ranges consisting of any upper limit or preferred value of the range and the lower limit or preferred value of the range have been specifically disclosed herein, regardless of whether these ranges are disclosed separately. In addition, if a numerical range is mentioned herein, unless otherwise specified, the range should include its endpoints and all integers and fractions within the range.

In this application, under the premise of achieving the purpose of the invention, numerical values should be understood to have the accuracy of the number of significant digits of the numerical value. For example, the number 40.0 should be understood to cover the range from 39.50 to 40.49.

The present invention is further described below in conjunction with examples. It should be noted that the following examples are provided for illustrative purposes only and do not constitute a limitation on the scope of protection claimed for the present invention.

Unless otherwise specified, the experimental methods used in the examples are all conventional methods.

Unless otherwise specified, the materials and reagents used in the examples can be obtained from commercial sources.

### Material:

The *Sargassum fusiforme* was purchased from Bozhou Chinese herbal medicine decoction pieces Company and was produced in Zhejiang, China.

### Equipment:

High performance gel permeation chromatography was performed using Shodex SUGAR KS-804 (8.0 mm × 300 mm, Agilent, USA) and Shodex SUGAR KS-802 (8.0 mm × 300 mm, Agilent, USA) columns in series, and a standard curve was prepared using T-series standard dextran (Dextran) with different molecular weights;
High performance liquid chromatography (HPLC) was performed using an Agilent 1260 Seri high performance liquid chromatography system (Agilent, USA);
Infrared analysis was performed using a Perkin-Elmer 599B infrared spectrophotometer (Perkin-Elmer, USA);
The nuclear magnetic resonance analysis was performed using a Brucker AM-500 nuclear magnetic resonance spectrometer (Brucker, Germany).

### Preparation Example 1: Preparation of seaweed-derived sulfated polysaccharide SPW-05-S2

### a. Extraction of Sargassum fusiforme sulfated polysaccharides:

2 kg of dried *Sargassum fusiforme* was added with 20 L × 2 deionized water, and extracted with boiling water at 100°C for 4 h each time, for a total of 8 times. The extracts were combined and concentrated, and dialyzed with running water for 3 days. The dialyzed liquid was heated and concentrated to 2.5 L, centrifuged and the precipitate was discarded, and the supernatant was added with 5 times the volume (12.5 L) of 95% v/v ethanol under stirring, and the alcohol precipitation was allowed to stand overnight, and centrifuged. The obtained precipitate was washed alternately with anhydrous ethanol and acetone for 3 times, and the precipitate was taken by centrifuging and dried under vacuum at 50°C, and then redissolved in 1500 mL deionized water, and freeze-dried to obtain 204 g of crude water-extracted *Sargassum fusiforme* sulfated polysaccharide (yield 10.2%).

### b. Sulfated polysaccharide purification:

6 g of the crude water-extracted *Sargassum fusiforme* sulfated polysaccharide from the above preparation was dissolved in 100 mL of deionized water, and the insoluble matter was removed by centrifugation at 4000 r/min for 10 min. The supernatant was separated by DEAE Sepharose Fast Flow anion exchange column, and gradient eluted with deionized water, 0.05 M, 0.1 M, 0.2 M, 0.3 M, 0.5 M, 0.6 M and 0.8 M NaCl solution in sequence, detected by sulfuric acid-phenol, and the elution curve was drawn. According to the elution curve, the 0.5 M NaCl eluate was collected and combined, concentrated, centrifuged, and the supernatant was dialyzed and freeze-dried to obtain about 508 mg of the initially purified sulfated polysaccharide SPW-05 (yield 8.46%). 200 mg of SPW-05 was dissolved in 4 mL of 0.2 M NaCl solution, centrifuged at 4000 r/min for 10 min, and the supernatant was eluted with 0.2 M NaCl solution through a Sephacryl HR S-300 gel chromatography column at a flow rate of 5 mL/15 min. The elution curve was drawn by detection with sulfuric acid-phenol method, and the sulfated polysaccharide SPW-05-S2 components were collected and combined according to the elution curve, concentrated, dialyzed, and freeze-dried to obtain about 166 mg of *Sargassum fusiforme* sulfated polysaccharide SPW-05-S2 (yield 32.7%).

### c. Structural identification and analysis of Sargassum fusiforme sulfated polysaccharide SPW-05-S2:

(1) The relative molecular mass of sulfated polysaccharide SPW-05-S2 was determined to be 121.77 kDa by high-performance gel permeation chromatography (HPGPC), and its purity was 95% (as shown in Figure 1, the small peak in the figure is the solvent peak).
   The completely hydrolyzed sample of sulfated polysaccharide SPW-05-S2 was measured by the PMP derivatization method to determine the monosaccharide composition, and it was determined that the sulfated polysaccharide SPW-05-S2 mainly contained mannose, glucuronic acid, galactose, xylose and fucose, with a molar ratio of 12.28:2.60:27.59:7.06:50.47. Methylation analysis was conducted. The raw sugar, desulfurized polysaccharide and reduced desulfurized polysaccharide were methylated and analyzed by GC-MS to determine the sugar residue connection mode and sulfate substitution position of the sulfated polysaccharide SPW-05-S2, and the connection mode of glucuronic acid was determined to be 1,4- glucuronic acid. The main chain of the sulfated polysaccharide SPW-05-S2 was determined to be 1,4-glucuronic acid and 1,2-mannose through the sugar composition and sugar residue connection mode of the secondary polysaccharide after partial acid hydrolysis. In addition, elemental analysis found that the sulfur content of the sulfated polysaccharide SPW-05-S2 was 9.76, and its sulfate content was further determined to be 29.28%. Using glucose as the standard, the sugar content was determined by the phenol-sulfuric acid method, indicating that the sugar content of the sulfated polysaccharide SPW-05-S2 was 66.4% (the average value of two sugar content determinations). In addition, the BCA kit detected that it also contained 3.4% protein.
(2) Infrared spectrum (as shown in Figure 2), the peak at 3308.69 cm⁻¹ is the O-H stretching vibration absorption peak, the peak at 2932.46 cm⁻¹ is the C-H stretching vibration absorption peak, the strong absorption peak at 1217.11 cm⁻¹ is the O=S=O stretching vibration peak, and the peak at 1732.38 cm⁻¹ is the carboxyl C=O stretching vibration, indicating that the polysaccharide contains sulfate and uronic acid.
(3) NMR analyze:
   35 mg of sulfated polysaccharide SPW-05-S2 was taken and added with 0.5 mL of D₂O to dissolve, 2.5 µL of acetone was added as internal standard (δH = 2.29 ppm, δC = 31.5 ppm). The one-dimensional and two-dimensional NMR spectra at 25°C were determined on a Bruker AVANCE III 500 M NMR instrument. The structure of sulfated polysaccharide SPW-05-S2 was confirmed by referring to the NMR spectrum. The ¹³C NMR result of SPW-05-S2 is shown in Figure 3.

In the ¹³C NMR spectrum of sulfated polysaccharide SPW-05-S2, in the anomeric carbon region, the signals of δ 105.48-δ 104.08 are attributed to 1,3,6-β-galactose, 1,6-β-galactose, and terminal β-galactose. The signals of δ 103.24 -δ 100.51 are attributed to 1,2,3-α-mannose/1,2,6-α-mannose, 1,4-β-xylose, terminal β-xylose, and 1,4-β-glucuronic acid. δ 99.69 -δ 97.44 are attributed to 1,2-α-fucose, 1,4-α-fucose, 1,2,4-α-fucose and terminal-α-fucose. δ 93.44 is attributed to 1,3-α-fucose. The δ 175.02 signal is attributed to the carboxyl carbon of 1,4-β-glucuronic acid. The signals of δ 39.47 and δ 21.83 indicate that the polysaccharide contains acetyl groups. δ 17.97 and δ 17.12 are attributed to C6 of α-fucose.

In the ¹H-QA (quantitative) NMR spectrum (Figure 4, without acetone), the signal at δ 5.0-5.7 is the signal of the anomeric hydrogen in the sulfated polysaccharide SPW-05-S2, the signal at δ 3.25-4.65 is the signal of the hydrogen on the sugar ring, the signal near δ 2.28 is the typical signal of the methyl hydrogen on the acetyl group, and the signal near δ 1.30 is the signal of fucose H6. According to the integrated area ratio of the signal near δ 2.28 and the signal near δ 1.30, the molar ratio of acetyl to fucose is 0.028:1. Since the molar fraction of fucose in the total sugar is 50.47 %, it can be converted to a molar ratio of acetyl to total sugar of 0.014:1.

The above results show that the *Sargassum fusiforme* sulfated polysaccharide SPW-05-S2 may have 1,4-β-glucuronic acid and 1,2-α-mannose as the main chain structure, and there are branches at the C-6 and C-3 positions of α-mannose, which are composed of galactose residues (1,3,6-β-galactose, 1,6-β-galactose and terminal-β-galactose), xylose (1,4-β-xylose and terminal-β-xylose) and fucose (1,2-α-fucose, 1,3-α-fucose, 1,4-α-fucose, 1,2,4-α-fucose and terminal-α-fucose) residues. By comparing the connection patterns of sugar residues before and after desulfurization, it was determined that the sulfate groups were mainly substituted at the C-4 position of 1,2-α-fucose and the C-2 position of 1,4-α- fucose, as well as the C-4 position of terminal-β-galactose and the C-4 position of partial terminal-β-xylose.

### Test Example 2: Anti-pulmonary fibrosis activity of seaweed-derived sulfated polysaccharide SPW-05-S2

### (1) Effects of Sargassum fusiforme polysaccharide component SPW-05-S2 on lung tissue morphology and lung tissue coefficient in mice were tested using bleomycin-induced pulmonary fibrosis model

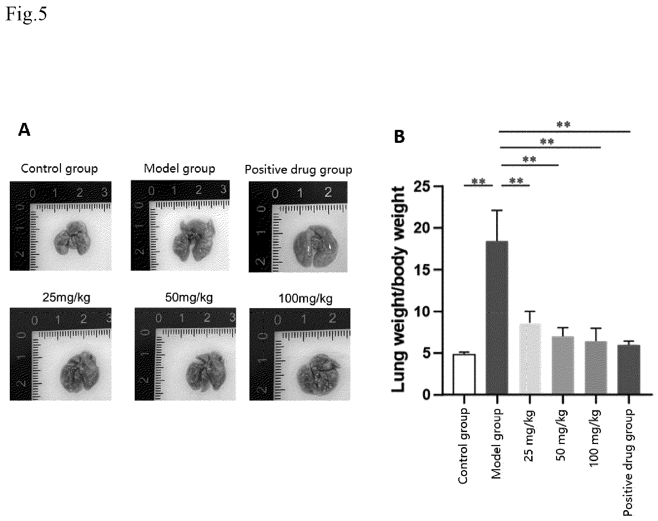

This animal experiment complied with the Guidelines for the Care and Use of Laboratory Animals and was approved by the Animal Ethics Committee of the Shanghai Institute of Materia Medica, Chinese Academy of Sciences. 36 of 8-week-old male C57BL/6 mice were purchased from Shanghai Slake Laboratory Animal Center Co., Ltd. The animals were randomly divided into three groups, 6 in the control group, 6 in the model group, and 24 in the experimental group. They were given free food and water, the room temperature was 22±1°C, the indoor relative humidity was 65±5%, and the light cycle was 12 h. In the model group and the experimental group, 1.8 mg/kg of bleomycin solution (purchased from CSNpharm) was administered intratracheally (prepared with 0.9% saline). Six mice were administered an equal volume of 0.9% saline intratracheally as the normal control group. One week later, the experimental group was randomly divided and administered, 6 mice in each group, into four groups, A, B, C, and D. Group A was given 25 mg/kg SPW-05-S2 polysaccharide, Group B was given 50 mg/kg SPW-05-S2 polysaccharide, Group C was given 100 mg/kg SPW-05-S2 polysaccharide, and Group D was given 100 mg/kg pirfenidone (purchased from MCE) as a positive drug control. The drugs were administered by gavage every day for two weeks. The control group and the model group were given an equal volume of saline. After two weeks of administration, the weight of the mice was recorded and the mice were sacrificed. The morphology of the lung tissue of the mice in each group was observed and the lung tissue was weighed. The results are shown in A and B in Figure 5. After the intervention of SPW-05-S2 polysaccharide, the surface of the lung tissue of the mice with pulmonary fibrosis was restored to a smooth and ruddy state with a bright color, but the texture was not as soft and elastic as the lung tissue of the mice in the control group. The lung coefficient (lung tissue weight/mouse body weight) of mice in the model group increased, while the lung coefficients of the low (25 mg/kg), medium (50 mg/kg), and high (100 mg/kg) dose groups of SPW-05-S2 decreased, which was significantly different from the model group.

### (2) Hematoxylin-eosin staining and Masson staining to observe the effect of SPW-05-S2 on alleviating pulmonary fibrosis

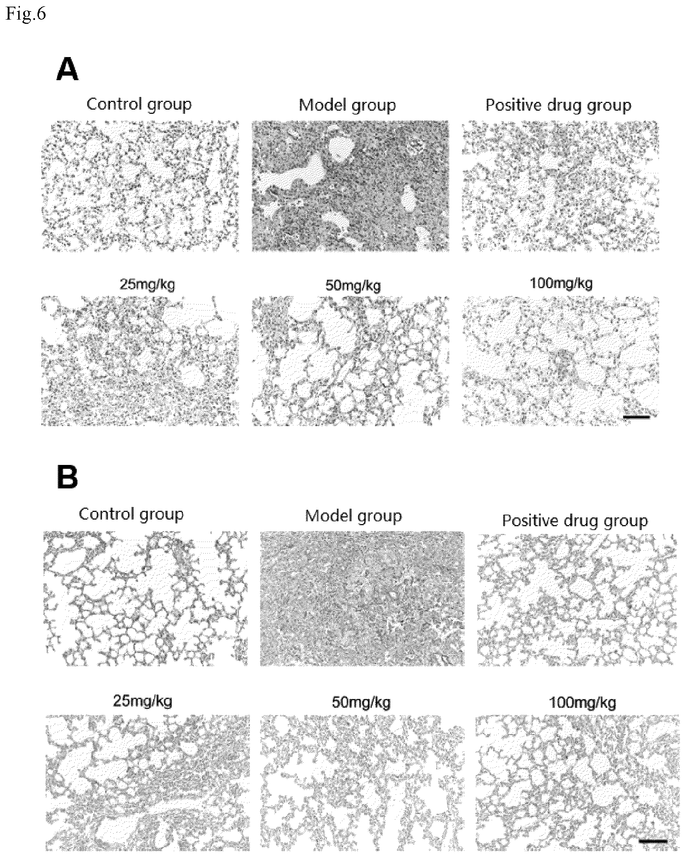

Two weeks after administration, the mice were sacrificed, and the left lung lobe was fixed with 4% paraformaldehyde for 48 hours, and then hematoxylin-eosin staining and Masson staining were performed. As shown in A and B in Figure 6, the results of hematoxylin-eosin staining showed that the degree of lung tissue lesions in mice in the low, medium and high dose groups of SPW-05-S2 was significantly reduced, and only a small number of interstitial vascular dilation, congestion and mild alveolitis were observed, but the degree of alveolar wall fibrosis was greatly reduced. The alveolar septum basically returned to normal, and no diffuse inflammatory infiltration was observed. The results of Masson staining showed that the alveolar integrity in the lung tissue of mice in the low, medium and high dose groups of SPW-05-S2 improved, the fibrous tissue clumps were significantly reduced, and the inhibition of collagen deposition was more obvious. It shows that the polysaccharide SPW-05-S2 can effectively alleviate lung fibrosis lesions, reduce fibrosis and slow down the process of lung fibrosis.

### (3) Western blotting assay to detect the effect of SPW-05-S2 on the levels of proteins related to fibrosis phenotype in mouse lungs

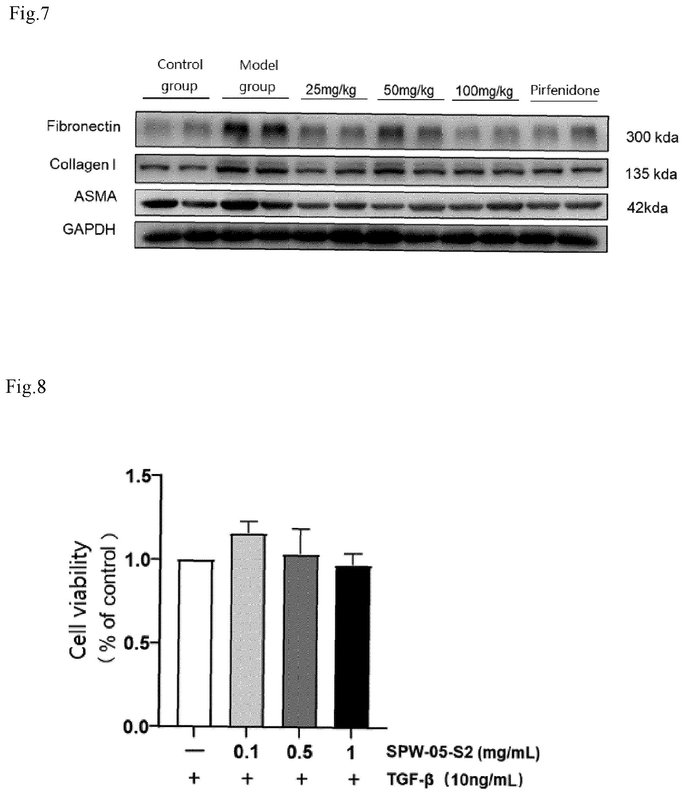

Two weeks after administration, the mice were sacrificed, and part of the lung tissue was extracted with a tissue extraction kit (purchased from Shenggong Biotechnology Co., Ltd.) to extract the protein. After adding 5× loading buffer, the protein was denatured at 95°C in a metal heater for 10 min, and then stored at -80°C after cooling. Immunoblotting was used to detect the expression of Fibronectin, Collagen I and α-smooth muscle actin proteins. The results are shown in Figure 7, the *Sargassum fusiforme* polysaccharide SPW-05-S2 can inhibit the expression of fibrosis phenotype-related proteins (Fibronectin, Collagen I and α-smooth muscle actin).

### (4) CCK-8 assay to detect the effect of Sargassum fusiforme polysaccharide SPW-05-S2 on the growth activity of alveolar epithelial cells A549

A549 cells (5×10³ cells/well) in the logarithmic growth phase were seeded into 96-well plates, set up three replicates, and cultured in an incubator for 24 h; the cell supernatant was aspirated, and the *Sargassum fusiforme* polysaccharide SPW-05-S2 solution with a final concentration of 0.1, 0.5 and 1 mg/mL was added. After culturing for 1 hour, TGF-β (10 ng/mL) was added for intervention treatment. After continuing to culture for 48 hours, 10 µL of CCK-8 enhanced solution (purchased from Dalian Meilun Company) was added to each well. After continuing to culture for 1 hour, the absorbance was detected at 450 nm using an enzyme reader. Cell growth activity was calculated according to the following formula: Cell activity = (OD value of the experimental group - OD value of the blank group) / (OD value of the control group - OD value of the blank group) × 100%. The results are shown in Figure 8, which indicates that SPW-05-S2 has no effect on cell growth activity (+ represents addition, - represents no addition).

### (5) Western blotting assay to detect the effect of Sargassum fusiforme polysaccharide SPW-05-S2 on the expression of fibrosis phenotype-related proteins in alveolar epithelial cells A549

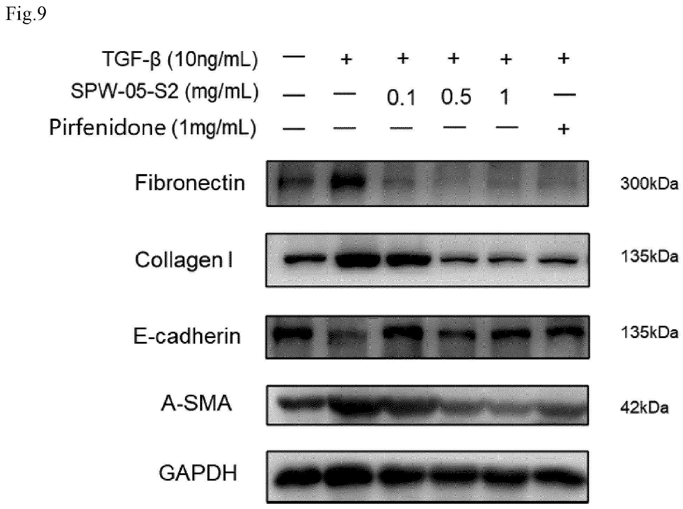

Human alveolar epithelial cells A549 were cultured in RPMI1640 medium containing 10% fetal bovine serum (purchased from Gibco, USA), 100 U/mL penicillin and 100 µg/mL streptomycin in a volume ratio of 1:1. A549 cells in the logarithmic growth phase were seeded in a 6-well plate at a density of 1.2×10⁶ cells/well. After culturing for 24 hours, they were treated with 0.1, 0.5 and 1 mg/mL *Sargassum fusiforme* polysaccharide SPW-05-S2 and positive drug 1mg/mL pirfenidone for 1 hour, and then stimulated by adding TGF-β recombinant protein (purchased from Abclonal) at a concentration of 0 and 10 ng/ml. After culturing for 24 hours, the supernatant was discarded, the cells were rinsed with pre-cooled PBS, and the cells were lysed on ice for 20 minutes with RIPA cell lysis buffer (purchased from Absin), and the supernatant was collected by centrifugation. After adding 5× loading buffer, the protein was denatured in a metal heater for 10 min, cooled and stored at -80°C. Immunoblotting was used to detect the protein expression of E-cadherin, α-smooth muscle actin, Collagen I and Fibronectin. The results are shown in Figure 9, *Sargassum fusiforme* polysaccharide SPW-05-S2 can significantly inhibit the expression of α-smooth muscle actin, Collagen I and Fibronectin proteins, and significantly increase the expression of E-cadherin protein.

In summary, it can be seen from the results of the test examples that, *Sargassum fusiforme* sulfated polysaccharide SPW-05-S2 can reduce bleomycin-induced pulmonary fibrosis, including restoring lung tissue morphology, reducing lung coefficient, and reducing the expression of animal lung injury and fibrosis phenotype-related proteins. In addition, it does not affect the cell growth viability of alveolar epithelial cells A549 in vitro. SPW-05-S2 can significantly inhibit the expression of fibrosis-related proteins α-SMA, Collagen I and Fibronectin, and significantly increase the expression of E-cadherin protein. Therefore, the polysaccharide component SPW-05-S2 can become a potential carbohydrate drug for preventing and/or treating pulmonary fibrosis.

The above examples are only used to illustrate the technical solutions of the present invention, rather than to limit them. Although the present invention has been described in detail with reference to the above examples, those skilled in the art should understand that the technical solutions described in the above examples may be modified, or some or all of the technical features thereof may be replaced by equivalents, without departing from the spirit and essence of the claims of the present invention; and these modifications or replacements are still within the scope defined by the claims of the present invention.

## Claims

1. A *Sargassum fusiforme* sulfated polysaccharide, **characterized in that**, by weight percentage, the *Sargassum fusiforme* sulfated polysaccharide contains 62% to 70% polysaccharide, sulfate group 27%~32%, acetyl 0.5%~1.5% and protein 2.0%~4.5%;
the polysaccharide is mainly composed of mannose, glucuronic acid, galactose, xylose and fucose.

2. The *Sargassum fusiforme* sulfated polysaccharide according to claim 1, **characterized in that** the structural unit of the polysaccharide is a main chain of 1→4-linked β-glucuronic acid and 1→2-linked α-mannose, with branches at the C-6 and C-3 positions of the α-mannose, and the branches are composed of 1,3,6-β-galactose, 1,6-β-galactose, terminal-β-galactose, 1,4-β-xylose, terminal-β-xylose, 1,2-α-fucose, 1,3-α-fucose, 1,4-α-fucose, 1,2,4-α-fucose and terminal-α-fucose residues;
the sulfate group is mainly substituted at the C-4 position of 1,2-α-fucose, the C-2 position of 1,4-α-fucose, the C-4 position of terminal-β-galactose, and the C-4 position of part of terminal-β-xylose.

3. The *Sargassum fusiforme* sulfated polysaccharide according to claim 1, **characterized in that** the weight average molecular weight of the *Sargassum fusiforme* sulfated polysaccharide is in the range of 5-1000 kDa, preferably 10-500 kDa, and more preferably 15-400 kDa.

4. The *Sargassum fusiforme* sulfated polysaccharide according to claim 1, **characterized in that** the molar ratio of mannose, glucuronic acid, galactose, xylose and fucose is 10-15:1.5-4:22-32:5-9:40-60, preferably 12.28:2.60:27.59:7.06:50.47.

5. The preparation method of *Sargassum fusiforme* sulfated polysaccharide according to any one of claims 1 to 4, **characterized in that** it comprises the following steps:
a. sulfated polysaccharide extraction:
extracting the dried *Sargassum fusiforme* with boiling water, the obtained extract is concentrated, dialyzed, re-concentrated, and centrifuged to remove the precipitate, the obtained supernatant is precipitated with alcohol, centrifuged and separated, and the obtained precipitate is washed and dried to obtain crude water-extracted *Sargassum fusiforme* sulfated polysaccharide;
b. sulfated polysaccharide purification:
b1. taking the crude water-extracted *Sargassum fusiforme* sulfated polysaccharide prepared in step a, dissolving in water and centrifuging. The supernatant was initially fractional purified by anion exchange column, sequentially eluted with water and 0.05 to 0.8 M NaCl solution, and the eluted fraction of about 0.5 M NaCl solution was collected to obtain sulfated polysaccharide SPW-05;
b2. dissolving the sulfated polysaccharide SPW-05 prepared in step b1 in about 0.01 to 1 times the weight of 0.2 M NaCl solution, centrifuging, and purifying the supernatant by gel chromatography column, eluting with 0.2 M NaCl solution, and collecting and combining the sulfated polysaccharide fractions.

6. The preparation method according to claim 5, **characterized in that**:
the step a comprises: adding 15 to 20 times the weight of water to the dried *Sargassum fusiforme,* soaking overnight, extracting with boiling water for 3 to 5 hours, 3 times a day, until there is no obvious color change detected by phenol sulfuric acid method; combining the extracts, concentrating, dialyzing, re-concentrating, centrifuging to obtain a supernatant, adding 5 to 10 times the volume of ethanol to the supernatant, centrifuging to obtain a precipitate, washing the precipitate with anhydrous ethanol and acetone for 3 to 6 times, and drying to obtain a crude water-extracted *Sargassum fusiforme* sulfated polysaccharide;
the step b comprises: b1. taking the crude water-extracted *Sargassum fusiforme* sulfated polysaccharide prepared in step a, adding about 10 to 20 times the weight of water to dissolve, centrifuging, separating the supernatant through an anion exchange column, eluting with water, 0.05 M, 0.1 M, 0.2 M, 0.3 M, 0.5 M, 0.6 M and 0.8 M NaCl solution in sequence, detecting with sulfuric acid-phenol, collecting and combining the eluate of about 0.5 M NaCl solution according to the elution curve, concentrating, centrifuging, dialyzing the supernatant, and freeze-drying to obtain the sulfated polysaccharide SPW-05;
b2. dissolving the sulfated polysaccharide SPW-05 prepared in step b1 in about 0.01 to 1 times the weight of 0.2 M NaCl solution, centrifuging, and purifying the supernatant by gel chromatography column, eluting with 0.2 M NaCl solution, detecting with a sulfuric acid-phenol method, and collecting and combining the component of the sulfated polysaccharide according to the elution curve, concentrating, dialyzing, and freeze-drying to obtain the sulfated polysaccharide.

7. The preparation method according to claim 6, **characterized in that** in step a, the ethanol is an ethanol aqueous solution of 70% (v/v) or more, preferably an ethanol aqueous solution of 85% (v/v) or more, and especially an ethanol aqueous solution of 95 % (v/v) or more.

8. A pharmaceutical composition comprising the *Sargassum fusiforme* sulfated polysaccharide according to any one of claims 1 to 4 and optionally a pharmaceutically acceptable excipient.

9. Use of the *Sargassum fusiforme* sulfated polysaccharide according to any one of claims 1 to 4 or the pharmaceutical composition according to claim 8 in the preparation of a medicament for preventing and/or treating pulmonary fibrosis.

10. The use according to claim 9, wherein the pulmonary fibrosis is bleomycin-induced pulmonary fibrosis.
